(19) Europäisches Patentamt / European Patent Office / Office européen des brevets

(11) **EP 3 804 682 A1**

(12) **EUROPEAN PATENT APPLICATION**
published in accordance with Art. 153(4) EPC

(43) Date of publication:
**14.04.2021 Bulletin 2021/15**

(51) Int Cl.:
**A61F 13/56** *(2006.01)*    **A61F 13/62** *(2006.01)*

(21) Application number: **19811325.0**

(86) International application number:
**PCT/JP2019/018699**

(22) Date of filing: **10.05.2019**

(87) International publication number:
**WO 2019/230331 (05.12.2019 Gazette 2019/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2018  JP 2018101633**

(71) Applicant: **Daio Paper Corporation**
**Ehime 799-0492 (JP)**

(72) Inventor: **NAGANO, Akiko**
**Shikokuchuo-shi, Ehime 799-0431 (JP)**

(74) Representative: **Williams Powell**
**11 Staple Inn**
**London WC1V 7QH (GB)**

(54)    **TAPE-TYPE DISPOSABLE DIAPER**

(57)    To suppress a wrinkle in a part from a non-connecting portion to a base portion of a connecting tape.

The problem is solved by a tape-type disposable diaper in which a lower edge 84L of a non-connecting portion 84 of a connecting tape 80 has a shape in which a tangent angle θ decreases without having a critical point and an inflection point from a distal end 84f far from a base portion 81 of the connecting tape 80 to a proximal end 84n close to the base portion 81, a tangent angle θ of the distal end 84f of the lower edge 84L of the non-connecting portion 84 is 80 to 85 degrees, a tangent angle θ of the proximal end 84n of the lower edge 84L of the non-connecting portion 84 is 0 degree, and when a shorter one of a distance in a width direction WD between the proximal end 84n of the lower edge 84L of the non-connecting portion 84 and the distal end 84f of the lower edge 84L of the non-connecting portion 84 and a distance in a front-back direction LD between the proximal end 84n of the lower edge 84L of the non-connecting portion 84 and the distal end 84f of the lower edge 84L of the non-connecting portion 84 is set to r, the entire lower edge 84L of the non-connecting portion 84 exclusively includes a part having a radius of curvature of 0.8r or more.

[Fig.8]

**Description**

Technical Field

The present invention relates to a tape-type disposable diaper.

Background Art

There are three main types of disposable diapers, which are tape-type, pants-type, and pad-type. Of these types of disposable diapers, the tape-type disposable diaper is worn by applying the tape-type disposable diaper to a body in an unfolded state and then connecting connecting portions provided on both right and left sides of a dorsal side part to an outer surface of a ventral side part.

A general tape-type disposable diaper has a crotch portion including a center in a front-back direction, a ventral side part extending to a front side of the center in the front-back direction, and a dorsal side part extending to a back side of the center in the front-back direction, at least the dorsal side part has wing parts extending to both right and left sides in a width direction from the crotch portion, the ventral side part and the dorsal side part have intermediate portions located between the right and left wing parts, and the wing parts have connecting portions detachably connected to the outer surface of the ventral side part. During use, the wing parts are turned from both sides of a waist to the outer surface of the ventral side part, and the connecting portions of the wing parts are connected to the outer surface of the ventral side part. Such a tape-type disposable diaper is used not only for infants but also for nursing care (adult).

Conventionally, as a method of providing the connecting portions on the wing parts, it is common to attach connecting tapes having the connecting portions to the wing parts of the dorsal side part. Each of the connecting tapes generally has a base portion fixed to the wing part, a main unit section extending from the base portion, a connecting portion provided in a middle of the main unit section in a width direction and to be detachably connected to the ventral side part, and a non-connecting portion provided between the connecting portion and the base portion and to be not connected to the ventral side part. (For example, see Patent Literatures 1 to 3). Note that for manufacturing reasons, in many products, an upper edge and a lower edge of the connecting portion correspond to an upper edge and a lower edge of the main unit section from a proximal end close to the base portion to a distal end far from the base portion, respectively.

Various shapes of the connecting tape have been devised, and it is preferable that the dorsal side part and the ventral side part are not easily shifted from each other, and the lower edge of the connecting tape has excellent fitting around legs. From such a point of view, the conventional connecting tape has a shape in which the lower edge of the main unit section is located on the upper side from the proximal end close to the base portion toward the distal end far from the base portion in many cases (For example, see Patent Literatures 1 to 3).

However, in the conventional tape-type disposable diaper including such a connecting tape, in a case where a size around legs of a wearer is larger than a product size to some extent (for example, near or above an upper limit of a conformity size of a product), or in a situation in which legs are pressed against the connecting tape as in the case of sitting, large wrinkles are apt to be formed in a part from the non-connecting portion to the base portion of the connecting tape, which may cause leakage and deterioration of wearing feeling.

Citation List

Patent Literature

Patent Literature 1: JP 2016-174816 A
Patent Literature 2: JP 2014-094197 A
Patent Literature 3: JP 11-155905 A

Summary of Invention

Technical Problem

Therefore, a main object of the invention is to suppress wrinkles in the part from the non-connecting portion to the base portion of the connecting tape, thereby suppressing leakage and deterioration of wearing feeling.

Solution to Problem

As a result of intensive research to solve the above problems, the inventor has found that in the conventional tape-type disposable diaper, the above problems are caused since the part from the non-connecting portion to the base portion of the connecting tape does not have a shape along a circumference of the legs, and is not easily deformed along the circumference of the legs in the situation in which the legs are pressed against the connecting tape. The following aspects solve the above problems based on such findings.

<First Aspect>

A tape-type disposable diaper including
a ventral side part extending to a front side of a center in a front-back direction, a dorsal side part extending to a back side of the center in the front-back direction, and a crotch portion extending from a middle of the ventral side part in the front-back direction to a middle of the dorsal side part in the front-back direction,
an absorber incorporated in a range including the crotch portion,

the dorsal side part having wing parts extending to outer sides of the crotch portion in a width direction, and connecting tapes attached to the wing parts of the dorsal side part and to be detachably connected to the ventral side part,

each of the connecting tapes having a base portion fixed to one of the wing parts, a main unit section extending from the base portion, a connecting portion provided in a middle of the main unit section in the width direction and to be detachably connected to the ventral side part, and a non-connecting portion provided between the connecting portion and the base portion and to be not connected to the ventral side part,

a lower edge of the main unit section having a shape in which a position of the lower edge shifts upward from a proximal end close to the base portion toward a distal end far from the base portion,

a lower edge of the connecting portion coinciding with the lower edge of the main unit section from the proximal end close to the base portion to the distal end far from the base portion,

in which a lower edge of the non-connecting portion has a shape in which a tangent angle decreases without having a critical point and an inflection point from a distal end far from the base portion toward a proximal end close to the base portion,

the lower edge of the non-connecting portion has a curved part at least in a part,

a tangent angle of a distal end of the lower edge of the non-connecting portion is 80 to 85 degrees, and a tangent angle of a proximal end of the lower edge of the non-connecting portion is 0 degree, and

when a shorter one of a distance in the width direction between the proximal end of the lower edge of the non-connecting portion and the distal end of the lower edge of the non-connecting portion and a distance in the front-back direction between the proximal end of the lower edge of the non-connecting portion and the distal end of the lower edge of the non-connecting portion is set to r, the entire lower edge of the non-connecting portion exclusively includes a part having a radius of curvature of 0.8r or more.

(Function and Effect)

In a state where the tape-type disposable diaper is worn, when the leg is pressed against the connecting tape, while the connecting portion is pushed up along a circumference of the leg, the non-connecting portion and the base portion are pulled from the connecting portion so as to be located on the lower side as the portions are away from the connecting portion. As a result, a tensile force acts on the lower edge of the non-connecting portion so that the distal end and the proximal end are separated from each other along the circumference of the leg, and a curved part at the lower edge of the non-connecting portion attempts deformation so as to approach a straight line.

Here, when a direction change of the lower edge of the non-connecting portion of the connecting tape is as large as 90 degrees, or a curved part or a bent part having a small curvature is included as in the conventional tape-type disposable diaper, a slight elongation of a material of the connecting tape is not enough to bring this part close to a straight line, and it is impossible to inhibit a large wrinkle from being formed in a part from the non-connecting portion to the base portion.

On the other hand, in this tape-type disposable diaper, the lower edge of the non-connecting portion is previously inclined at the distal end so that the tangent angle is 80 to 85 degrees, which is along the circumference of the leg. From there, the tangent angle decreases toward the proximal end where the tangent angle is 0 degrees, without having a critical point and an inflection point. Moreover, the entire lower edge of the non-connecting portion exclusively includes the portion having the radius of curvature of 0.8r or more, so that the tangent angle decreases particularly gently. If a sufficient inclination occurs at the distal end as the lower edge of the non-connecting portion, and the most part thereof gently bends, when a tensile force in an around-leg direction acts on the lower edge of the non-connecting portion, slight elongation of the material of the connecting tape accumulates, so that the curved part easily approaches a straight line. Therefore, as compared with the conventional one, the large wrinkle is less likely to be formed in the part from the non-connecting portion to the base portion. In addition, when the lower edge of the non-connecting portion is sufficiently inclined at the distal end and the most part thereof gently bends, fitting around the legs becomes excellent even in a state before extending in a direction along the circumference of the leg.

Note that the "tangent angle" refers to an angle formed by a tangent line (itself in the straight line part) and the front-back direction on the front side and the base portion side. In addition, "without having a critical point and an inflection point" means in other words that there is continuity in the change in curvature. The lower edge of the non-connecting portion may include a linear part as well as a curved part. The curvature of the straight line in this case is 0, and the radius of curvature is infinite.

<Second Aspect>

The tape-type disposable diaper according to the first aspect,

in which the distance in the width direction between the proximal end of the lower edge of the non-connecting portion and the distal end of the lower edge of the non-connecting portion is 1.0 to 1.5 times the distance in the front-back direction between the proximal end of the lower edge of the non-connecting portion and the distal end of the lower edge of the non-connecting portion.

(Function and Effect)

When the lower edge of the non-connecting portion has the length and width equal to each other or is horizontally long, a wrinkle suppressing property and fitting around the legs become particularly excellent, which is preferable.

<Third Aspect>

The tape-type disposable diaper according to the first or second aspect,
in which the connecting portion is a part to which a hook member of a mechanical fastener is attached, and
the lower edge of the non-connecting portion includes a linear part linearly extending from the distal end to a side of the proximal end and a curved part extending from the linear part to the proximal end, a width direction dimension of the linear part being 1 to 5 mm.

(Function and Effect)

By having such a linear part, the corner of the hook member is unlikely to become unnecessarily sharp, which is preferable. Further, when the dimension of the linear part is 5 mm or less, the curved part can have a sufficiently large and gentle shape.

<Fourth Aspect>

The tape-type disposable diaper according to any one of the first to third aspects,
in which a front-back direction dimension of the lower edge of the non-connecting portion from a position where a tangent angle is 3 degrees to the proximal end is 20% or less of a front-back direction dimension of the non-connecting portion.

(Function and Effect)

As described above, by sufficiently shortening a part having a small tangent angle (that is, close in the front-back direction) in the vicinity of the proximal end in the lower edge of the non-connecting portion, the wrinkle suppressing property and fitting around the legs become particularly excellent, which is preferable.

<Fifth Aspect>

The tape-type disposable diaper according to any one of the first to fourth aspects,
in which the non-connecting portion has an elongation of 1.1 to 1.3 times when pulled in the width direction at 20 N/25 mm.

(Function and Effect)

It is good that the non-connecting portion is a material that easily stretches. However, when the non-connecting portion excessively easily stretches, the diaper is more likely to slip due to body movement after wearing. For this reason, it is preferable that the elongation property of the non-connecting portion falls within the above range.

<Sixth Aspect>

The tape-type disposable diaper according to any one of the first to fifth aspects, further including side flap portions not having the absorber on both sides in the width direction from the ventral side part to the dorsal side part,
in which a concave edge from a side edge of the crotch portion to a lower edge of the wing part is formed by cutting a middle in the front-back direction in a side portion of one of the side flap portions, and
when a tangent line common to the concave edge and the lower edge of the non-connecting portion is drawn, a front-back direction dimension from a contact point of the tangent line at the lower edge of the non-connecting portion to the proximal end is 45% or less of the front-back direction dimension of the non-connecting portion.

(Function and Effect)

In this aspect, in a situation where the legs are pressed against the connecting tapes, breaks or wrinkles are rarely formed in the non-connecting portion. In addition, in this aspect, fitting around the legs of a part, which is from the concave edge formed on the side flap portion to the non-connecting portion of the connecting tape becomes excellent.

<Seventh Aspect>

The tape-type disposable diaper according to any one of the first to sixth aspects,
in which the connecting tapes have line symmetry with respect to a center line passing through the center in the front-back direction, and an upper half edge and a lower half edge thereof are point-symmetrical with respect to center points located at the center in the front-back direction and a center in the width direction, respectively.

(Function and Effect)

When the connecting tapes have a shape as in this aspect, the connecting tapes having bilateral symmetry can be efficiently manufactured by a known method that does not generate waste materials, which is preferable.

Advantage Effects of Invention

The invention is advantageous in that wrinkles are suppressed in the part from the non-connecting portion to the base portion of the connecting tape.

Brief Description of Drawings

Fig. 1 is a plan view illustrating an inner surface of a tape-type disposable diaper in a state where a diaper is spread.
Fig. 2 is a plan view illustrating an external surface of the tape-type disposable diaper in a state where the diaper is spread.
Fig. 3 is a cross-sectional view taken along line 6-6 in Fig. 1.
Fig. 4 is a cross-sectional view taken along line 7-7 in Fig. 1.
Fig. 5(a) is a cross-sectional view taken along line 8-8 in Fig. 1, and Fig. 5(b) is a cross-sectional view taken along line 9-9 in Fig. 1.
Fig. 6 is a cross-sectional view taken along line 5-5 in Fig. 1.
Fig. 7 is an enlarged plan view of a part having a connecting tape.
Fig. 8 is an enlarged view of a main part of the part having the connecting tape, in which Fig. 8(a) is a plan view of an unfolded state, and Fig. 8(b) is a plan view of a deformed state.
Fig. 9 is an enlarged plan view of a part having a connecting tape.
Fig. 10 is an enlarged view of a main part of the part having the connecting tape, in which Fig. 10(a) is a plan view of an unfolded state, and Fig. 10(b) is a plan view of a deformed state.
Fig. 11 is a plan view schematically illustrating a method of manufacturing the connecting tape.
Fig. 12 is a plan view of the unfolded state in which the main part of the part having the connecting tape is enlarged and illustrated.

Description of Embodiments

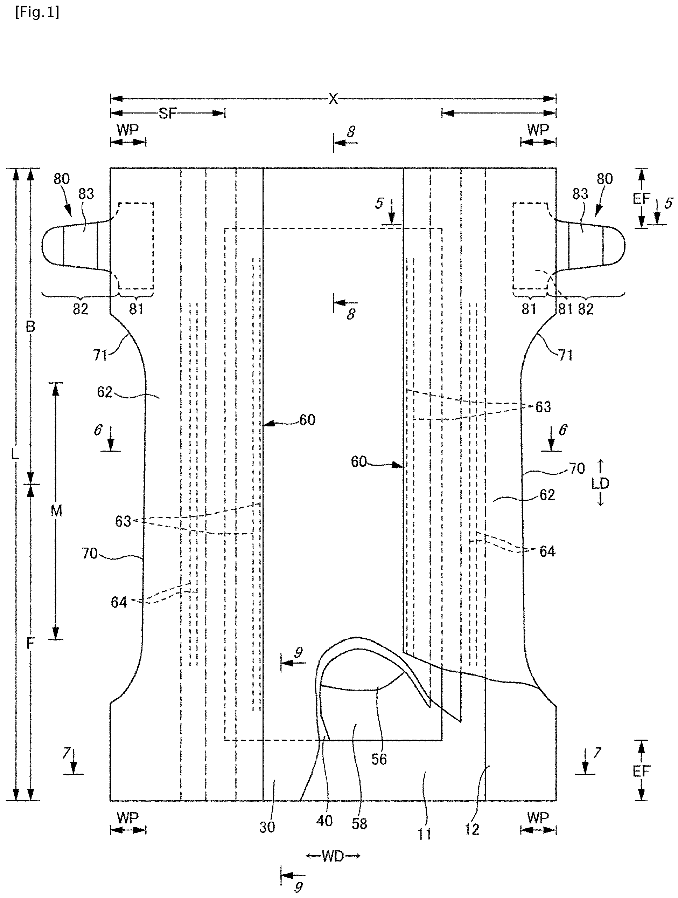

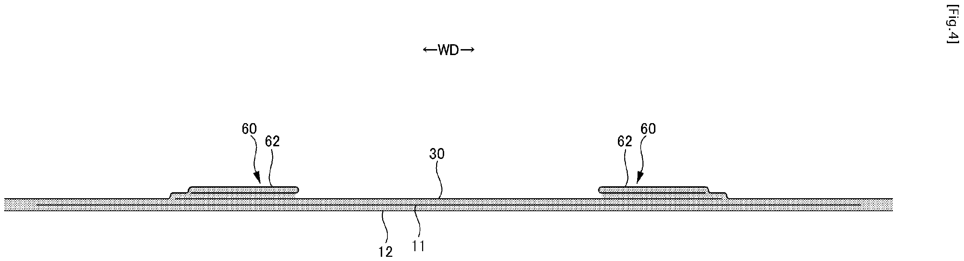

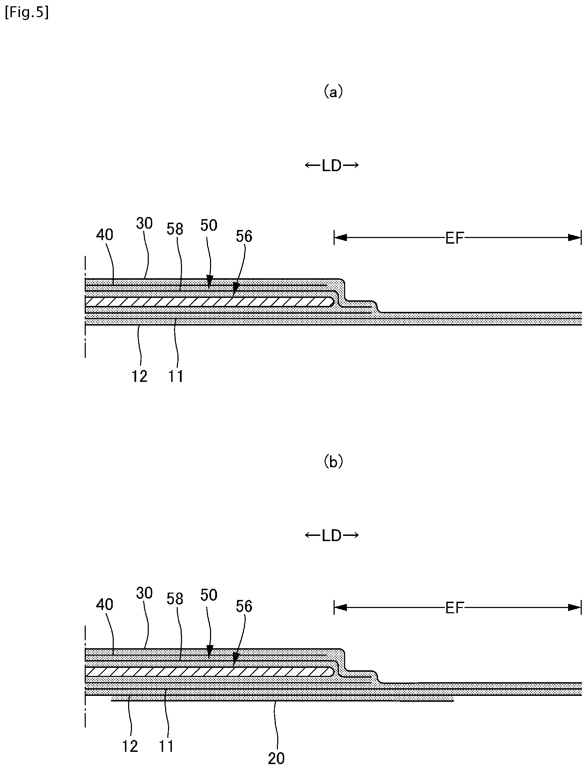

Figs. 1 to 6 illustrate an example of a tape-type disposable diaper. In the figures, a reference character X indicates a maximum width of the diaper excluding a connecting tape, a reference character L indicates a maximum length of the diaper, and a dotted pattern portion in cross-sectional views indicates a hot melt adhesive as bonding means that bonds respective constituent members located on a front surface side and a back surface side thereof. The hot melt adhesive can be applied by a known method such as slot application, continuous linear or dotted bead application, spiral or Z-shaped spray application, or pattern application (transfer of the hot melt adhesive in a letterpress method). Instead or additionally, in a fixing part of an elastic member, the hot melt adhesive can be applied to an outer peripheral surface of the elastic member, and the elastic member can be fixed to an adjacent member. Examples of the hot melt adhesive include EVA-based, pressure sensitive adhesion rubber-based (elastomer-based), polyolefin-based, and polyester/polyamide-based adhesives, and can be used without any particular limitation. As bonding means that bonds respective components, it is possible to use means by material welding such as heat sealing or ultrasonic sealing.

This tape-type disposable diaper has a ventral side part F extending forward from a center in the front-back direction LD and a dorsal side part B extending backward from the center in the front-back direction LD. In addition, this tape-type disposable diaper has an absorber 56 incorporated in a range including a crotch portion, a liquid pervious top sheet 30 that covers the front surface side of the absorber 56, a liquid impervious sheet 11 that covers the back surface side of the absorber 56, and an outer nonwoven fabric 12 that covers the back surface side of the liquid impervious sheet and forms an outer surface of a product.

Materials and characteristic parts of respective portions will be described below in order.

(Absorber)

The absorber 56 is a part that absorbs and retains excreted liquid, and can be formed of a fiber assembly. As this fiber assembly, in addition to those obtained by accumulating short fibers such as fluff pulp and synthetic fibers, it is possible to use a filament assembly obtained by opening a tow (fiber bundle) of synthetic fibers such as cellulose acetate as required. A fiber basis weight can be set to, for example, about 100 to 300 g/m$^2$ in the case of accumulating fluff pulp or short fibers, and can be set to, for example, about 30 to 120 g/m$^2$ in the case of the filament assembly. The fineness in the case of the synthetic fiber is, for example, 1 to 16 dtex, preferably 1 to 10 dtex, and more preferably 1 to 5 dtex. In the case of the filament assembly, the filament may be a non-crimped fiber, and is preferably a crimped fiber. A crimp degree of the crimped fiber can be set to, for example, about 5 to 75, preferably 10 to 50, and more preferably about 15 to 50 per 2.54 cm. In addition, crimped fibers uniformly crimped can be used.

(Super Absorbent Polymer Particle)

The absorber 56 may contain super absorbent polymer particles in a part or all thereof. The super absorbent polymer particles include "powder" in addition to "particles". As the super absorbent polymer particles, those used for this type of absorbent articles can be used on an as-is basis. A particle size of the super absorbent polymer particle is not particularly limited. For example, it is desirable to use those in which when sieving (shaking for 5 minutes) using a standard sieve of 500 μm (JIS Z8801-1:2006) and sieving (shaking for 5 minutes) using a standard sieve (JIS Z8801-1:2006) of 180 μm for particles falling under the former sieve are performed, a ratio of particles remaining on the standard sieve of 500 μm is 30% by weight or less, and a ratio of particles remaining on the standard sieve of 180 μm is 60% by weight or more.

The material of the super absorbent polymer particles can be used without particular limitation, but the material having the water absorption capacity of 40 g/g or more is suitable. As the super absorbent polymer particles, starch-based, cellulose-based, and synthetic polymer-based, and starch-polyacrylate (salt) graft copolymers, saponified starch-acrylonitrile copolymers, sodium carboxymethyl cellulose crosslinked products, polyacrylate (salt) polymers, and the like can be used. As the shape of the super absorbent polymer particles, the shapes of particulate materials which are usually used are suitable, but other shapes can also be used.

The super absorbent polymer particles having a water absorption rate of 70 seconds or less, particularly 40 seconds or less, are suitably used. If the water absorption rate is too slow, returning, in which the liquid fed into the absorber 56 returns to the outside of the absorber 56, is likely to occur.

In addition, the super absorbent polymer particles having the gel strength of 1,000 Pa or more are preferably used. Thereby, even when the absorber 56 is bulky, it is possible to effectively suppress stickiness after liquid absorption.

The basis weight of the super absorbent polymer particles can be appropriately determined according to the absorption amount required for the use of the absorber 56. Therefore, although it cannot be said unconditionally, in a normal case, the basis weight can be 50 to 350 g/m$^2$.

(Wrapping Sheet)

In order to prevent the super absorbent polymer particles from slipping out or to improve the shape retention of the absorber 56, the absorber 56 can be incorporated as an absorbent element 50 wrapped with a wrapping sheet 58. As the wrapping sheet 58, tissue paper, particularly crepe paper, a nonwoven fabric, a polyethylene laminated nonwoven fabric, a sheet with small holes, and the like can be used. However, it is desirable that the wrapping sheet be a sheet through which the super absorbent polymer particles do not pass. When a nonwoven fabric is used in place of the crepe paper, a hydrophilic SMMS (spun bond/melt blown/melt blown/spun bond) nonwoven fabric is particularly suitable, and polypropylene, polyethylene/polypropylene, etc. can be used as a material. The basis weight of the fiber is desirably 5 to 40 g/m$^2$, particularly desirably 10 to 30 g/m$^2$.

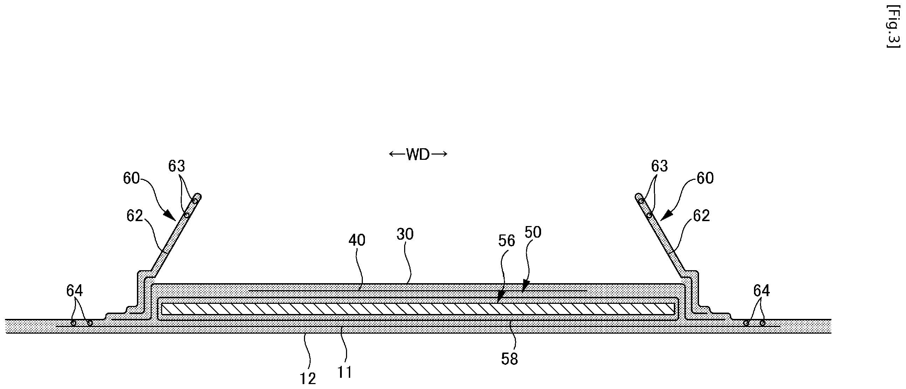

The wrapping sheet 58 may have a structure in which one sheet wraps the entire absorber 56 as illustrated in Fig. 3, or the entire absorber 56 may be wrapped with a plurality of sheets (two upper and lower sheets). The wrapping sheet 58 may be omitted.

(Top Sheet)

The top sheet 30 has a liquid pervious property, and for example, it is possible to use a porous or non-porous nonwoven fabric, a porous plastic sheet, etc. Moreover, of these materials, a raw material fiber of the nonwoven fabric is not particularly limited. Examples of the constituent fibers can include synthetic fibers such as polyolefin-based fibers such as polyethylene and polypropylene, polyester-based fibers, and polyamide-based fibers, regenerated fibers such as rayon and cupra, natural fibers such as cotton or the like, mixed fibers and composite fibers in which two or more of these are used, and the like. Further, the nonwoven fabric may be manufactured by any processing. Examples of processing methods can include known methods such as a spun lace method, a spun bond method, a thermal bond method, a melt blown method, a needle punch method, an air through method, and a point bond method. For example, the spun lace method is preferable when flexibility and drapeability are required, and the thermal bond method is preferable when bulkiness and softness are required.

The top sheet 30 extends from a front end to a back end of the product in the front-back direction, and extends to a lateral side of the absorber 56 in the width direction WD. However, for example, when starting points of rising gathers 60 described later are located on a center side of side edges of the absorber 56 in the width direction, it is possible to make appropriate modification such as making a width of the top sheet 30 shorter than the maximum width of the absorber 56 as necessary.

(Intermediate Sheet)

To rapidly transfer a liquid permeating the top sheet 30 to the absorber, it is possible to provide an intermediate sheet (also referred to as "second sheet") 40 having a higher liquid permeation rate than that of the top sheet 30. The intermediate sheet 40 is intended to rapidly transfer the liquid to the absorber to improve the absorption performance of the absorber and prevent a "returning" phenomenon of the absorbed liquid from the absorber. The intermediate sheet 40 can be omitted.

Examples of the intermediate sheet 40 can include the same material as that of the top sheet 30, a spunlace nonwoven fabric, a spunbond nonwoven fabric, an SMS nonwoven fabric, a pulp nonwoven fabric, a mixed sheet of pulp and rayon, a point bond nonwoven fabric, or crepe paper. In particular, an air through nonwoven fabric is bulky, and thus is preferable. It is preferable to use a composite fiber having a core-sheath structure for the air through nonwoven fabric. In this case, a resin used for the core may be polypropylene (PP), and is preferably polyester (PET) having high rigidity. A basis weight is preferably 17 to 80 g/m$^2$, more preferably 25 to 60 g/m$^2$. A thickness of a raw material fiber of the nonwoven fabric is preferably 2.0 to 10 dtex. In order to make the nonwoven fabric bulky, it is preferable to use eccentric fibers having no core in the center, hollow fibers, or eccentric and hollow fibers as mixed fibers of all or some of raw material fibers.

Although the intermediate sheet 40 in the illustrated example is shorter than the width of the absorber 56 and disposed at the center portion, it may be provided over the maximum width. Further, the intermediate sheet 40 may be provided over the maximum length of the diaper, and may be provided only at an intermediate part including an excretion position as in the illustrated example.

(Liquid Impervious Sheet)

The liquid impervious sheet 11 is not particularly limited, and preferably has moisture permeability. As the liquid impervious sheet 11, for example, a microporous sheet can be preferably used which is obtained by kneading a polyolefin-based resin such as polyethylene or polypropylene and an inorganic filler, molding the kneaded materials into a sheet and monoaxially or biaxially stretching the sheet. Further, as the liquid impervious sheet 11, it is possible to use a sheet containing a nonwoven fabric as a base material and having enhanced waterproofness.

It is desirable that the liquid impervious sheet 11 extends in the front-back direction LD and the width direction WD in the same or wider range as or than that of the absorber 56. However, in a case where other water blocking means are present, etc., as necessary, it is possible to adopt a structure in which an end portion of the absorber 56 is not covered in the front-back direction LD and the width direction WD.

(Outer Nonwoven Fabric)

The outer nonwoven fabric 12 covers the entire back surface side of the liquid impervious sheet 11 and makes the outer surface of the product look like a cloth. The outer nonwoven fabric 12 is not particularly limited. As a raw material fiber, for example, in addition to synthetic fibers such as polyolefin-based fiber such as polyethylene or polypropylene, polyester-based fiber, and polyamide-based fiber, regenerated fibers such as rayon and cupra, and natural fibers such as cotton can be used. As a processing method, a spun lace method, a spun bond method, a thermal bond method, an air through method, a needle punch method, and the like can be used. However, a long-fiber nonwoven fabric such as a spunbonded nonwoven fabric, an SMS nonwoven fabric, and an SMMS nonwoven fabric is preferable in that good texture and strength can be compatible. In addition to using a single piece of nonwoven fabric, it is also possible to use multiple nonwoven fabrics in layers. In the latter case, it is preferable that the nonwoven fabrics are adhered to each other with a hot melt adhesive or the like. When a nonwoven fabric is used, the basis weight of the fiber is desirably 10 to 50 g/m$^2$, particularly desirably 15

to 30 g/m$^2$.

(Rising Gather)

In order to block excrement that moves laterally on the top sheet 30 and prevent so-called side leakage, the rising gathers 60 standing up to the skin side of the wearer are preferably provided on both sides of the surface in the width direction WD. Naturally, the rising gathers 60 can be omitted.

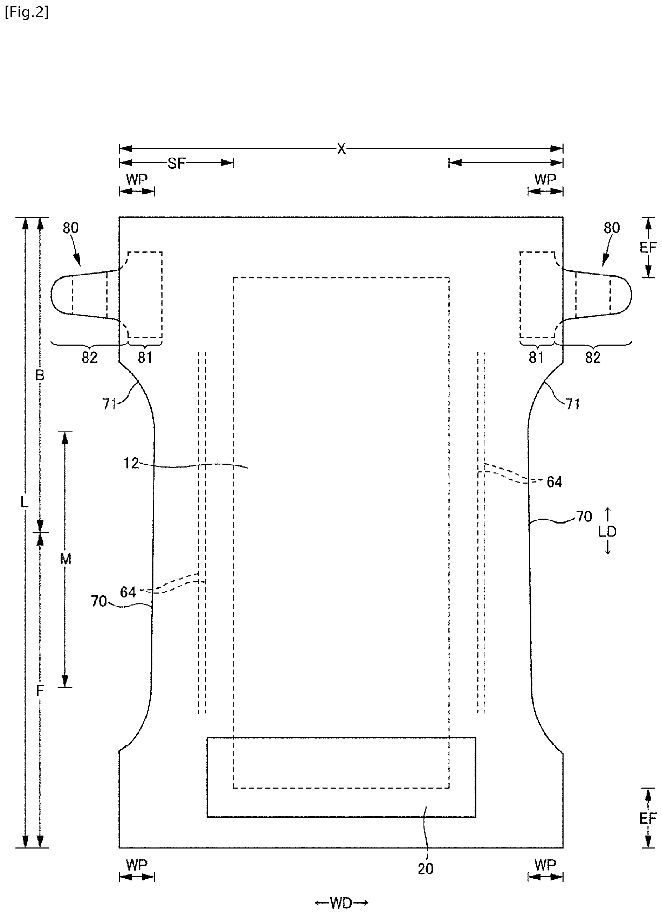

When the rising gathers 60 are adopted, a structure thereof is not particularly limited, and any known structure can be adopted. Each of the rising gathers 60 of the illustrated example includes a gather sheet 62 that is substantially continuous in the width direction WD and an elongated gather elastic member 63 fixed to the gather sheet 62 in a stretched state along the front-back direction LD. As this gather sheet 62, a water repellent nonwoven fabric can be used, and rubber thread or the like can be used as the gather elastic member 63. As illustrated in Figs. 1 and 2, a plurality of the elastic members may be provided on each side, or only one elastic member may be provided on each side.

The inner surface of the gather sheet 62 has a joint start point in the width direction WD on the side portion of the top sheet 30, and a part from the joint start point to an outer side in the width direction is bonded to an inner surface of each side flap portion SF, that is, a side portion of the liquid impervious sheet 11 and a side portion of the outer nonwoven fabric 12 located on the outer side thereof in the width direction in the illustrated example by a hot melt adhesive, etc.

In the circumference of the leg, the inside in the width direction from the joint start point of each rising gather 60 is fixed on the top sheet 30 at both ends of the product in the front-back direction. However, the portion therebetween is a non-fixed free portion, and this free portion is erected by contraction force of the elastic member 63 and comes into close contact with a body surface.

(End Flap Portion and Side Flap Portion)

The tape-type disposable diaper of the illustrated example includes a pair of end flap portions EF that extends to the front side and the back side of the absorber 56, respectively, and does not have the absorber 56, and a pair of side flap portions SF that extends laterally beyond both side edges of the absorber 56 and does not have the absorber 56. The side flap portions SF may be made of a material (outer nonwoven fabric 12, etc.) continuous from a portion having the absorber 56 as in the illustrated example, or may be formed by attaching another material.

(Flat Gather)

Side elastic members 64 made of an elongated elastic member such as rubber thread, etc. are fixed to the respective side flap portions SF in a state of being extended along the front-back direction LD. In this way, around-leg parts of the respective side flap portions SF are configured as flat gathers. The side elastic members 64 may be provided between the gather sheet 62 and the liquid impervious sheet 11 on the outer side in the width direction near the joint start point in the bonded portion of the gather sheet 62 as in the illustrated example, or may be provided between the liquid impervious sheet 11 and the outer nonwoven fabric 12 in the side flap portions SF. As in the illustrated example, a plurality of side elastic members 64 may be provided on each side, or only one side elastic member 64 may be provided on each side.

The flat gather is a part where the contraction force of the side elastic members 64 acts (the part where the side elastic members 64 are illustrated in the figure). Therefore, in addition to a mode in which the side elastic members 64 are present only in a part of the flat gather, the following structure is included. In this structure, even though the side elastic members 64 are present on the front side, back side, or both sides of the flat gather, the side elastic members 64 are finely cut at one place or a plurality of places in a part other than a site of the flat gather, are not fixed to sheets interposing the side elastic members 64 therebetween, or correspond to both the cases, so that the contraction force does not act on a site other than the flat gather (substantially equivalent to not providing the elastic members), and the contraction force of the side elastic members 64 acts only on the site of the flat gather.

(Wing Part)

In the tape-type disposable diaper, the dorsal side part B has wing parts WP extending to the outer side of the crotch portion M in the width direction WD. Similarly, the ventral side part F has wing parts WP extending to the outer side of the crotch portion M in the width direction WD. These wing parts WP can be formed by members different from the other parts. However, in the structure having the side flap portions SF as in the illustrated example, when a middle in the front-back direction LD in each of side portions of the side flap portions SF is cut to form a concave edge 70 from a side edge of the crotch portion M to a lower edge 71 of the wing part, and as a result the wing part WP is formed, it is easy to manufacture, and thus it is preferable.

(Connecting Tape)

As illustrated in Figs. 1, 2, and 6, connecting tapes 80 to be detachably connected to the outer surface of the ventral side part F are provided in the wing parts WP in the dorsal side part B, respectively. When the diaper is worn, the connecting tapes 80 are turned from both sides of the waist to the outer surface of the ventral side part F, and connecting portions 83 of the connecting tapes 80 are connected to proper positions on the outer surface of the ventral side part F.

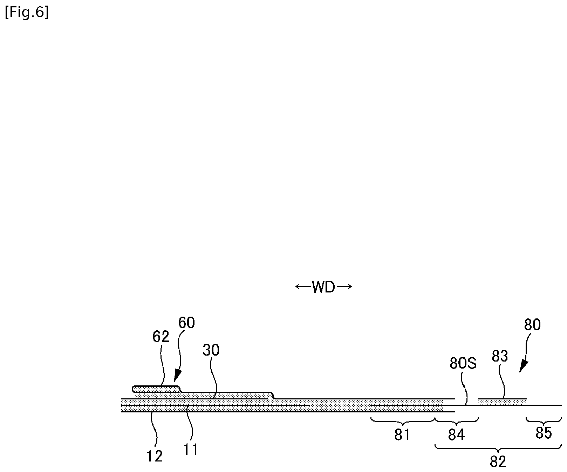

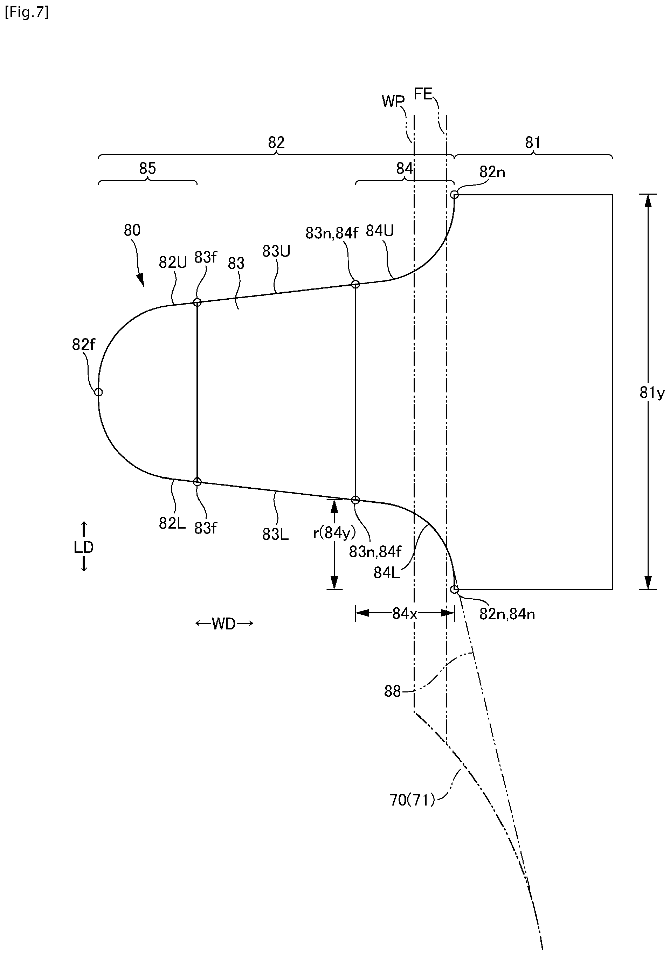

As illustrated in Figs. 6 and 7, each of the connecting tapes 80 has a base portion 81 fixed to the wing part WP, a sheet base material 80S forming a main unit section 82 extending from the base portion 81, and the connecting portion 83 for the ventral side part F provided at an intermediate portion of the main unit section 82 in the width direction WD in the sheet base material 80S. In the main unit section 82, a part on the base portion 81 side of the connecting portion 83 is a non-connecting portion 84 not connected to the ventral side part F, and a part on the opposite side is a tab part 85. The non-connecting portion 84 and the tab part 85 include only the sheet base material 80S forming the main unit section 82. As in most products, an upper edge 82U and a lower edge 82L of the main unit section 82 have a shape in which the upper edge 82U extends downward and the lower edge 82L extends upward from a proximal end 82n close to the base portion 81 toward a distal end 82f far from the base portion 81, respectively (in other words, a shape in which a dimension in the front-back direction LD shortens from the proximal end 82n to the distal end 82f). An upper edge 83U and a lower edge 83L of the connecting portion 83 have linear shapes in the illustrated example. However, the upper edge 83U and the lower edge 83L may have curved shapes. A tip of the tab part 85 has removed and rounded upper and lower corners as in most products. However, the invention is not limited thereto. A shape of the base portion 81 is a rectangular shape whose one side is the proximal end of the main unit section 82 as in most products. However, the invention is not limited thereto. The shape of the base portion 81 is generally a rectangular shape that is long in the front-back direction LD as in the illustrated example. However, the shape may be a rectangular shape that is long in the width direction WD or a square shape.

As the connecting portion 83, a hook member (male member) of a mechanical fastener (hook and loop fastener) may be provided, or an adhesive layer may be provided. The hook member has a plurality of engagement protrusions on a connecting surface thereof, and the engagement protrusion has a (A) check mark shape, a (B) J shape, a (C) mushroom shape, a (D) T shape, a (E) double J shape (a shape bonded back to back of a J shape), and the like, but may have any shape. The upper edge 83U and the lower edge 83L of the connecting portion 83 correspond to the upper edge 82U and the lower edge 82L of the main unit section 82 from the proximal end 83n close to the base portion 81 to the distal end 83f far from the base portion 81, respectively. Such a structure can be manufactured by a manufacturing method described later.

Further, as the sheet base material 80S forming from the base portion 81 to the main unit section 82, a nonwoven fabric, a plastic film, a polyethylene laminated nonwoven fabric, paper, or a composite material thereof can be used, and it is preferable to use a spunbonded nonwoven fabric, an air through nonwoven fabric, or a spunlace nonwoven fabric having a fineness of 1.0 to 3.5 dtex, a basis weight of 60 to 100 g/m², and a thickness of 1 mm or less.

In the connecting tape 80, a boundary FE between a fixed portion and a non-fixed portion with respect to the wing part WP preferably coincides with a boundary between the base portion 81 and the non-connecting portion 84 (position passing through the proximal end 82n). However, complete coincidence is difficult in manufacturing, and thus the boundary may be located at an end portion of the non-connecting portion 84 on the base portion 81 side or located at an end portion of the base portion 81 on the non-connecting portion 84 side. When the connecting tape 80 is interposed between a front surface side sheet (gather sheet 62 in the illustrated example) that covers a front surface side thereof and a back surface side sheet (outer nonwoven fabric 12 in the illustrated example) that covers a back surface side thereof, and fixed by a hot melt adhesive, etc. as in the illustrated example, a side edge of a bonded region of the front surface side sheet and the back surface side sheet becomes the boundary FE between the fixed portion and the non-fixed portion with respect to the wing part WP in the connecting tape 80. Note that as can be seen from this description, it is desirable that the entire base portion 81 of the connecting tape 80 is fixed to the wing part WP. However, only a part thereof may be fixed to the wing part WP. Similarly, it is desirable that the entire non-connecting portion 84 is not fixed to the wing part WP. However, only a part thereof may be fixed to the wing part WP. In addition, as illustrated in Fig. 6, a part or all of the non-connecting portion 84 may be located between the front surface side sheet (gather sheet 62 in the illustrated example) and the back surface side sheet (outer nonwoven fabric 12 in the illustrated example), and not be fixed to the front surface side sheet and the back surface side sheet.

(Target Sheet)

It is preferable to provide a target portion at a connecting position with the connecting tape 80 in the ventral side part F. The target portion can be provided by attaching a target sheet 20 for facilitating the connection to the outer surface of the ventral side part F as in the illustrated example. In a case where the connecting portion 83 is the hook member, it is possible to use the target sheet 20 having a large number of loops made of threads to which engagement protrusions of the hook member are tangled and is provided on a surface of the sheet base member made of a plastic film or a nonwoven fabric. Further, in the case of a pressure sensitive adhesion material layer, it is possible to use a sheet base material made of a plastic film having a smooth surface with a high pressure sensitive adhesion property and the surface of which is subjected to a release treatment. In addition, in a case where the connecting position of the connecting tape 80 in the ventral side part F is made of a nonwoven fabric, for example, in the case of having the outer nonwoven fabric 12 as in the illustrated example, the target sheet 20 may be omitted, and the hook member may be entwined with fibers of the outer nonwoven fabric 12 and connected. In this case, the target sheet 20 as a mark may be provided between the outer nonwoven fabric 12 and the liquid impervious sheet 11, or a mark may be provided on the outer surface of the outer nonwoven fabric 12 or the liquid impervious sheet 11.

(Shape of Connecting Tape)

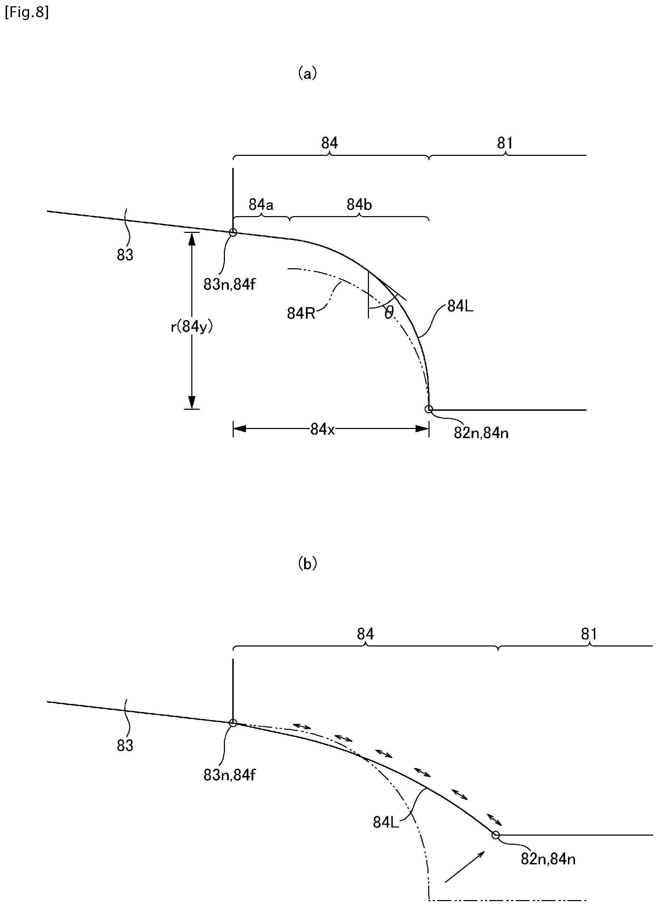

Characteristically, as illustrated in Fig. 8(a), a lower edge 84L of the non-connecting portion 84 has a shape in which a tangent angle θ decreases without having a critical point and an inflection point from a distal end 84f far from the base portion 81 toward a proximal end 84n close to the base portion 81. In addition, the tangent angle θ of the lower edge of the non-connecting portion 84 is 80 to 85 degrees at the distal end 84f and 0 degree at the proximal end 84n of the lower edge of the non-connecting portion 84. Further, when a shorter one of a distance 84x in the width direction WD between the proximal end 84n of the lower edge 84L of the non-connecting portion 84 and the distal end 84f of the lower edge 84L of the non-connecting portion 84 and a distance 84y in the front-back direction LD between the proximal end 84n of the lower edge 84L of the non-connecting portion 84 and the distal end 84f of the lower edge 84L of the non-connecting portion 84 is set to r, the entire lower edge 84L of the non-connecting portion 84 exclusively includes a part having a radius of curvature of 0.8r (0.8 times r) or more. Note that an arc of a chain double-dashed line indicated by reference character 84R in Fig. 8(a) is an arc of a radius 0.8r illustrated to compare radii of curvature. More preferably, the entire lower edge 84L of the non-connecting portion 84 is only a part having a radius of curvature of 0.85r (0.85 times r) or more.

In a state where the tape-type disposable diaper is worn, when the leg is pressed against the connecting tape 80, as illustrated in Fig. 8(b), while the connecting portion 83 is pushed up along a circumference of the leg, the non-connecting portion 84 and the base portion 81 are pulled from the connecting portion 83 so as to extend downward as the portions are away from the connecting portion 83. As a result, a tensile force acts on the lower edge 84L of the non-connecting portion 84 so that the distal end 84f and the proximal end 84n are separated from each other along the circumference of the leg, and a curved part at the lower edge 84L of the non-connecting portion 84 tends to deform so as to approach a straight line.

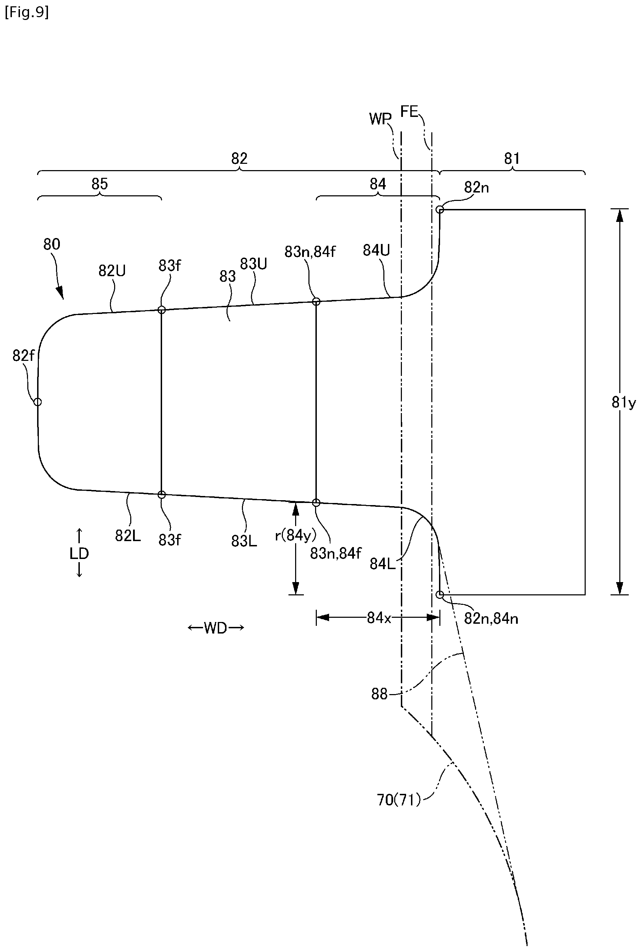

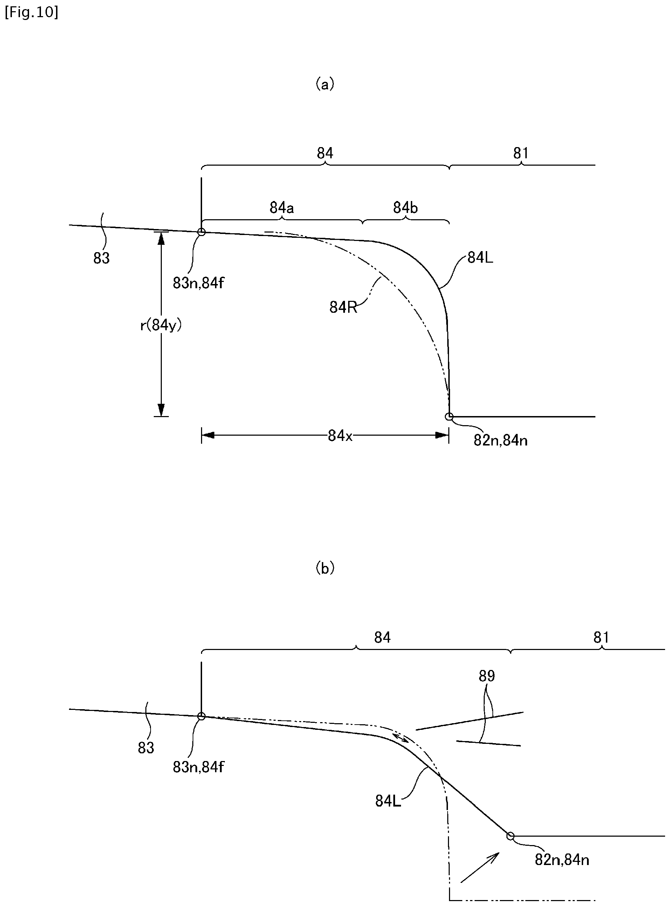

Here, when the lower edge 84L of the non-connecting portion 84 has a curved part 84b having a small curvature as in the conventional tape-type disposable diaper illustrated in Figs. 9 and 10(a), a slight elongation of a material of the connecting tape 80 is not enough to bring the curved part 84b close to a straight line as illustrated in Fig. 10(b), and it is impossible to inhibit a large wrinkle 89 from being formed in a part from the non-connecting portion 84 to the base portion 81. A similar result is obtained when the lower edge 84L of the non-connecting portion 84 has a bent part, or a direction change of the lower edge of the non-connecting portion 84 of the connecting tape 80 is as large as 90 degrees.

On the other hand, in this tape-type disposable diaper, as illustrated in Fig. 8(a), the lower edge 84L of the non-connecting portion 84 is previously inclined at the distal end 84f so that the tangent angle $\theta$ is 80 to 85 degrees, which is along the circumference of the leg. From there, the tangent angle $\theta$ decreases toward the proximal end 84n where the tangent angle $\theta$ is 0 degrees, without having a critical point and an inflection point. Moreover, the entire lower edge 84L of the non-connecting portion 84 exclusively includes the portion having the radius of curvature of 0.8r or more, so that the tangent angle $\theta$ decreases particularly gently. If a sufficient inclination occurs at the distal end 84f as the lower edge 84L of the non-connecting portion 84 and the most part thereof gently bends, when a tensile force in an around-leg direction acts on the lower edge 84L of the non-connecting portion 84 as illustrated in Fig. 8(b), slight elongation (indicated by arrows in the figure) of the material of the connecting tape 80 accumulates, so that the curved part easily approaches a straight line. Therefore, as compared with the conventional one, the large wrinkle 89 is less likely to be formed in the part from the non-connecting portion 84 to the base portion 81. In addition, when the lower edge 84L of the non-connecting portion 84 is sufficiently inclined at the distal end 84f and the most part thereof gently bends, fitting around the legs becomes excellent even in a state before extending in a direction along the circumference of the leg.

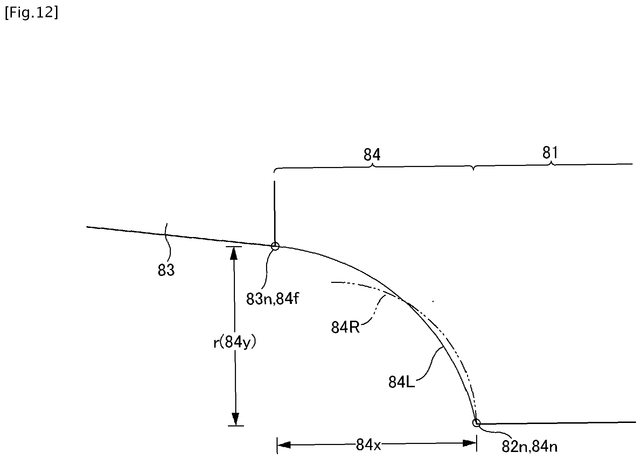

Note that, as described above, the "tangent angle $\theta$" refers to an angle formed by a tangent line (itself in the straight line part) and the front-back direction LD on the front side and the base portion 81 side (see Fig. 8(a)). In addition, "without having a critical point and an inflection point" means in other words that there is continuity in the change in curvature. The lower edge 84L of the non-connecting portion 84 can include a linear part 84a as well as the curved part 84b, as illustrated in Fig. 8(a), for example. The curvature of the straight line in this case is 0, and the radius of curvature is infinite. Naturally, as illustrated in Fig. 12, the lower edge 84L of the non-connecting portion 84 may be only the curved part.

A front-back direction dimension and a width direction dimension of the lower edge 84L of the non-connecting portion 84 can be appropriately determined. However, in a normal case, the front-back direction dimension and the width direction dimension are preferably 0.2 to 0.25 times a front-back direction dimension 81y of the base portion 81 and 0.2 to 0.3 times a width direction dimension of the main unit section 82, respectively. In addition, a distance 84x in the width direction WD between the proximal end 84n of the lower edge 84L of the non-connecting portion 84 and the distal end 84f of the lower edge 84L of the non-connecting portion 84 can be appropriately determined. However, when the distance 84x is 1.0 to 1.5 times a distance 84y in the front-back direction LD between the proximal end 84n of the lower edge 84L of the non-connecting portion 84 and the distal end 84f of the lower edge 84L of the non-connecting portion 84 (that is, the length and width are equal or the width is long), a wrinkle suppressing property and fitting around the legs become particularly excellent, which is preferable.

In recent years, the connecting portion 83 is formed of a hook member of a mechanical fastener in many cases. In such a case, as in the illustrated example, the lower edge 84L of the non-connecting portion 84 has the linear part 84a linearly extending from the distal end 84f to the proximal end 84n side and the curved part 84b extending from the linear part 84a to the proximal end 84n, and a width direction dimension of the linear part 84a is preferably 1 to 5 mm. By having such a linear part 84a, the corner of the hook member is unlikely to become unnecessarily sharp. Further, when the dimension of the linear part 84a is 5 mm or less, the curved part 84b can have a sufficiently large and gentle shape.

By sufficiently shortening a part having a small tangent angle $\theta$ (that is, close in the front-back direction LD) in the vicinity of the proximal end 84n in the lower edge 84L of the non-connecting portion 84, the wrinkle suppressing property and fitting around the legs become particularly excellent. For this reason, it is preferable that the lower edge 84L of the non-connecting portion 84 has a front-back direction dimension from a position where the tangent angle $\theta$ is 3 degrees to the proximal end 84n to be 20% or less of a front-back direction dimension of the non-connecting portion 84 (equal to the distance 84y). In the lower edge 84L of the non-connecting portion 84, the vicinity of the proximal end 84n may be a linear part.

In a case where the concave edge 70 from the side edge of the crotch portion M to the lower edge 71 of the wing part is formed by cutting the middle of the side portion of the side flap portion SF in the front-back direction LD as in the illustrated example, when a tangent line 88 common to the concave edge 70 and the lower edge 84L of the non-connecting portion 84 is drawn, a dimension in the front-back direction from a contact point of the tangent line 88 at the lower edge of the non-connecting portion 84 to the proximal end 84n is preferably 45% or less of the dimension of the non-connecting portion 84 in the front-back direction. In this way, in a situation where the legs are pressed against the connecting tapes 80, breaks or wrinkles are rarely formed particularly in the non-connecting portion 84. In addition, fitting around the legs of a part, which is from the concave edge 70 formed on the side flap portion SF to the non-connecting portion 84 of the connecting tape 80 becomes excellent.

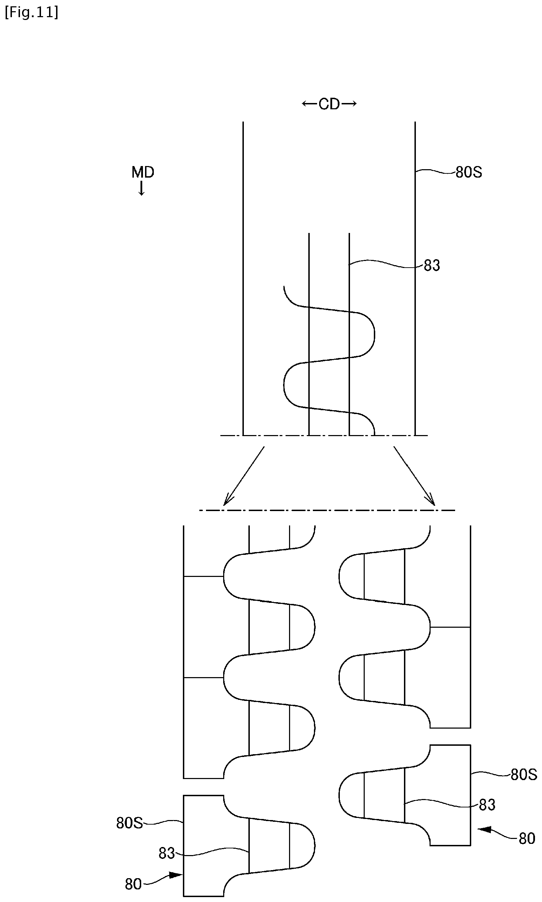

When the connecting tapes 80 have line symmetry with respect to a center line passing through the center in the front-back direction, and an upper half edge and a lower half edge thereof are point-symmetrical with respect to center points located at the center in the front-back direction and the center in the width direction, respectively, the connecting tapes 80 having bilateral symmetry can be efficiently manufactured by a known method that does not generate waste materials, which is preferable. That is, as illustrated in Fig. 11, while the continuous belt-shaped sheet base material 80S is transferred along a continuous direction, the connecting portions 83 are continuously provided along an MD (Machine Direction) on a center portion in a CD (Cross Direction). Then, while being reciprocally displaced in the CD so as to traverse the connecting portions 83, the sheet base material 80S is divided into two parts by periodic wavy lines extending in the MD and cut at a predetermined interval in the MD. In this way, it is possible to manufacture each of the connecting tape 80 on one of right and left sides from a division part on one side in the CD and the connecting tape 80 on the other side from the division part on the other side in the CD.

It is good that the non-connecting portion 84 is a material that easily stretches. However, when the non-connecting portion 84 excessively easily stretches, the diaper is more likely to slip due to body movement after wearing. Therefore, it is preferable that the non-connecting portion 84 has an elongation of 1.1 to 1.3 times, particularly 1.1 to 1.2 times when pulled in the width direction WD at 20 N/25 mm. In the case of the illustrated example, it is preferable to use a material having such an elongation property as the sheet base material 80S forming the main unit section 82 and the base portion 81. As a nonwoven fabric having such an elongation property, a nonwoven fabric having a basis weight of 60 to 100 $g/m^2$ is preferable. Note that the elongation of the non-connecting portion 84 is measured by the following method. That is, the connecting tape 80 is removed from the diaper, and both front and back parts of the base portion 81 are cut off along a straight line in the width direction WD passing through the distal end of the non-connecting portion 84 to produce a test piece. A width of the test piece is a minimum value of the dimension of the non-connecting portion 84 in the front-back direction. Thereafter, an interval of a gripping tool in a tensile testing machine is adjusted to the width direction dimension of the non-connecting portion 84 which is a test piece , the connecting portion 83 side and the base portion 81 side of the non-connecting portion 84 are gripped with the gripping tool, and a tensile test is conducted under a condition of a tensile speed of 100 mm/min. Based on a measurement result of the tensile test, the elongation is obtained by the following formula. Note that the following dimension i can be easily obtained by measuring the width of the test piece as 25 mm. However, when the width of the test piece is not 25 mm, the dimension can be obtained by converting the tensile force per 25 mm width.

$$\text{Elongation (times)} = i/i_0$$

i: Width direction dimension of the non-connecting portion 84 when pulled at 20 N/25 mm.
$i_0$: Width direction dimension of the non-connecting portion 84 at the beginning (before pulling).

<Description of Terms Used Herein>

The following terms in the description have the following meanings unless otherwise specified in the specification.

- "Front-back direction" means a direction (longitudinal direction) indicated by the reference character LD in the figure, "width direction" means a direction (right-left direction) indicated by WD in the figure, and the front-back direction and the width direction are orthogonal to each other.
- "Curved line" means not including a straight line.
- "Unfolded state" means a flatly spread state without contraction or slack.
- "Stretch rate" means the value when the natural length is taken as 100%. For example, a stretch rate of 200% is synonymous with a stretch magnification of 2 times.
- "Gel strength" is measured as follows: 1.0 g of super absorbent polymer is added to 49.0 g of artificial urine (urea: 2 wt%, sodium chloride: 0.8 wt%, calcium chloride dihydrate: 0.03 wt%, magnesium sulfate heptahydrate: 0.08 wt%, and ion exchanged water: 97.09 wt%), and stirred with a stirrer. The resulting gel is left for three hours in a thermohygrostat bath at 40°C×60%RH and then cooled to room temperature. The gel strength of the gel is measured with a curd meter (Curdmeter-MAX ME-500, manufactured by I.techno Engineering).
- "Basis weight" is measured as follows. After the sample or test piece is preliminary dried, it is allowed to stand in a test room or apparatus under normal conditions (the test location is at a temperature: 23 ± 1°C, relative humidity: 50 ± 2%) until the constant mass. The preliminary drying is to make the sample or test piece be constant mass in an environment of a temperature of 100°C. Note that the fibers of an official moisture regain of 0.0% do not need preliminary drying. From a test piece having a constant mass, a sample having a size of 100 mm × 100 mm is cut out using a template for sampling (100 mm × 100 mm). The sample is weighed and the weight is multiplied by 100 into the weight per one square meter. The resulting value is defined as the basis weight.

- "Thickness" is automatically measured under the conditions of a load of 0.098 N/cm$^2$ in a pressurized area of 2 cm$^2$ using an automatic thickness measuring device (KES-G5 handy compression tester).

- "Water absorption capacity" is measured in accordance with JIS K7223-1996 "Testing method for water absorption capacity of super absorbent resins".

- "Water absorption rate" is the "time that elapses before the end point" measured in accordance with JIS K7224-1996 "Testing method for water absorption rate of super absorbent resins" using 2 g of super absorbent polymer and 50 g of physiological saline solution.

- When environmental conditions in tests and measurements are not described, the tests and measurements shall be carried out in a test room or apparatus under normal conditions (the test location is at a temperature: 23 ± 1°C, relative humidity: 50 ± 2%).

- The dimension of each part means the dimension in the unfolded state, not the natural length state, unless otherwise specified.

Industrial Applicability

The invention is applicable to the tape-type disposable diaper as in the above example.

Reference Signs List

| | |
|---|---|
| 11 | LIQUID IMPERVIOUS SHEET |
| 12 | OUTER NONWOVEN FABRIC |
| 80 | CONNECTING TAPE |
| 83 | CONNECTING PORTION |
| 82 | MAIN UNIT SECTION |
| 81 | BASE PORTION |
| 20 | TARGET SHEET |
| 30 | TOP SHEET |
| 40 | INTERMEDIATE SHEET |
| 50 | ABSORBENT ELEMENT |
| 56 | ABSORBER |
| 58 | WRAPPING SHEET |
| 60 | RISING GATHER |
| 62 | GATHER SHEET |
| 64 | SIDE ELASTIC MEMBER |
| 70 | CONCAVE EDGE |
| 71 | LOWER EDGE OF WING PART |
| B | DORSAL SIDE PART |
| F | VENTRAL SIDE PART |
| LD | FRONT-BACK DIRECTION |
| M | CROTCH PORTION |
| SF | SIDE FLAP PORTION |
| WD | WIDTH DIRECTION |
| WP | WING PART |
| 80S | SHEET BASE MATERIAL |
| 84 | NON-CONNECTING PORTION |
| 85 | TAB PART |
| θ | TANGENT ANGLE |

89     WRINKLE

**Claims**

1. A tape-type disposable diaper comprising:

a ventral side part extending to a front side of a center in a front-back direction;
a dorsal side part extending to a back side of the center in the front-back direction;
a crotch portion extending from a middle of the ventral side part in the front-back direction to a middle of the dorsal side part in the front-back direction, the dorsal side part having wing parts extending to outer sides of the crotch portion in a width direction;
an absorber incorporated in a range including the crotch portion; and
connecting tapes attached to the wing parts of the dorsal side part and to be detachably connected to the ventral side part,
each of the connecting tapes having a base portion fixed to one of the wing parts, a main unit section extending from the base portion, a connecting portion provided in a middle of the main unit section in the width direction and to be detachably connected to the ventral side part, and a non-connecting portion provided between the connecting portion and the base portion and to be not connected to the ventral side part,
a lower edge of the main unit section having a shape in which a position of the lower edge shifts upward from a proximal end close to the base portion toward a distal end far from the base portion,
a lower edge of the connecting portion coinciding with the lower edge of the main unit section from the proximal end close to the base portion to the distal end far from the base portion,
wherein a lower edge of the non-connecting portion has a shape in which a tangent angle decreases without having a critical point and an inflection point from a distal end far from the base portion toward a proximal end close to the base portion,
the lower edge of the non-connecting portion has a curved part at least in a part,
a tangent angle of a distal end of the lower edge of the non-connecting portion is 80 to 85 degrees, and a tangent angle of a proximal end of the lower edge of the non-connecting portion is 0 degree, and
when a shorter one of a distance in the width direction between the proximal end of the lower edge of the non-connecting portion and the distal end of the lower edge of the non-connecting portion and a distance in the front-back direction between the proximal end of the lower edge of the non-connecting portion and the distal end of the lower edge of the non-connecting portion is set to r, the entire lower edge of the non-connecting portion exclusively includes a part having a radius of curvature of 0.8r or more.

2. The tape-type disposable diaper according to claim 1,
wherein the distance in the width direction between the proximal end of the lower edge of the non-connecting portion and the distal end of the lower edge of the non-connecting portion is 1.0 to 1.5 times the distance in the front-back direction between the proximal end of the lower edge of the non-connecting portion and the distal end of the lower edge of the non-connecting portion.

3. The tape-type disposable diaper according to claim 1 or 2,
wherein the connecting portion is a part to which a hook member of a mechanical fastener is attached, and
the lower edge of the non-connecting portion includes a linear part linearly extending from the distal end to a side of the proximal end and a curved part extending from the linear part to the proximal end, a width direction dimension of the linear part being 1 to 5 mm.

4. The tape-type disposable diaper according to any one of claims 1 to 3,
wherein a front-back direction dimension of the lower edge of the non-connecting portion from a position where a tangent angle is 3 degrees to the proximal end is 20% or less of a front-back direction dimension of the non-connecting portion.

5. The tape-type disposable diaper according to any one of claims 1 to 4,
wherein the non-connecting portion has an elongation of 1.1 to 1.3 times when pulled in the width direction at 20 N/25 mm.

6. The tape-type disposable diaper according to any one of claims 1 to 5, further comprising side flap portions not having the absorber on both sides in the width direction from the ventral side part to the dorsal side part,
wherein a concave edge from a side edge of the crotch portion to a lower edge of the wing part is formed by cutting a middle in the front-back direction in a side portion of one of the side flap portions, and when a tangent line common to the concave edge and the lower edge of the non-connecting portion is drawn, a front-back direction dimension from a contact point of the tangent line at the lower edge of the non-connecting portion to the proximal end is 45% or less of the front-back direction dimension of the non-connecting portion.

7. The tape-type disposable diaper according to any one of claims 1 to 6,
wherein the connecting tapes have line symmetry with respect to a center line passing through the center in the front-back direction, and an upper half edge and a lower half edge thereof are point-symmetrical with respect to center points located at the center in the front-back direction and a center in the width direction, respectively.

[Fig.1]

[Fig.2]

[Fig.3]

[Fig.4]

[Fig.5]

(a)

←LD→

(b)

←LD→

[Fig.6]

←WD→

EP 3 804 682 A1

[Fig.7]

20

[Fig.8]

(a)

(b)

[Fig.9]

[Fig.10]

(a)

(b)

[Fig.11]

[Fig.12]

**INTERNATIONAL SEARCH REPORT**

| International application No. |
|---|
| PCT/JP2019/018699 |

A. CLASSIFICATION OF SUBJECT MATTER
Int.Cl. A61F13/56(2006.01)i, A61F13/62(2006.01)i

According to International Patent Classification (IPC) or to both national classification and IPC

B. FIELDS SEARCHED

Minimum documentation searched (classification system followed by classification symbols)
Int.Cl. A61F13/56, A61F13/62

Documentation searched other than minimum documentation to the extent that such documents are included in the fields searched
Published examined utility model applications of Japan    1922–1996
Published unexamined utility model applications of Japan   1971–2019
Registered utility model specifications of Japan          1996–2019
Published registered utility model applications of Japan  1994–2019

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2017-23288 A (OJI HOLDINGS CORPORATION) 02 | 1–6 |
| Y | February 2017, paragraphs [0017], [0024]–[0025], fig. 3–4 (Family: none) | 7 |
| Y | JP 2008-161571 A (DAIO PAPER CORP.) 17 July 2008, paragraph [0041], fig. 6, 7 (Family: none) | 7 |

☐ Further documents are listed in the continuation of Box C.    ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 July 2019 (17.07.2019) | 30 July 2019 (30.07.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016174816 A
- JP 2014094197 A
- JP 11155905 A